# EUROPEAN PATENT APPLICATION

(11) **EP 4 563 074 A1**
(43) Date of publication of application: **04.06.2025**
(21) Application number: 23212753.0
(22) Date of filing: 28.11.2023
(51) Int. Cl.: A61B 5/00, A61B 5/021, A61B 5/024, A61B 5/08, A61B 5/11, A61B 5/1455, A61B 5/349, A61B 5/352, A61B 5/369, A61B 5/389, G16H 40/63, G16H 40/67

(54) **MODULAR SYSTEM AND METHOD FOR SYNCHRONIZATION OF MULTI-SENSOR HEALTH DATA COLLECTION**

(71) Applicant: AIKON Technologies B.V., 5656 AE Eindhoven (NL)
(72) Inventor: KANAGASABAPATHI, Thirukumaran Thangaraj, 5656 AE Eindhoven (NL); SCHULING, Franklin Harold, 5656 AE Eindhoven (NL); KJELLANDER, Birgitta Katarina Charlotte, 5656 AE Eindhoven (NL); PHOELICH, Pieter Franciscus, 5656 AE Eindhoven (NL); HIDALGO MONTANEZ, Brenda Sofia, 5656 AE Eindhoven (NL); PANDITHA, Venkata Krishna Pradeep, 5656 AE Eindhoven (NL)
(74) Representative: V.O.

(57) **Abstract**

A modular system and methods for collecting health-related data via a network of wearable devices is disclosed. The system has a hub that wirelessly connects to multiple wearable devices attachable to various body parts. These devices are capable of independently collecting health data, which can be synchronized by the hub when time-aligned measurements are needed. The hub sends trigger signals to the wearable devices to initiate and synchronize this data collection, ensuring accurate and timely health metrics. Additionally, the system supports bidirectional communication between the hub and devices for data transmission and synchronization in real-time. The methods described include configuring the modular system and the process of collecting synchronized health-related data using the hub and wearable devices network.

## Description

### FIELD OF THE INVENTION

The invention relates to a modular system comprising a hub and modular wearable devices wirelessly connectable to the hub. Furthermore, the invention relates to a method of configuring a modular system for collecting health-related data. The invention also relates to a method for collecting health-related data using a modular system. Additionally, the invention relates to a hub configured to wirelessly connect to a plurality of modular wearable devices.

### BACKGROUND TO THE INVENTION

In the field of wearable health technology, the accurate monitoring and analysis of health-related data is of great importance. The advent of wearable devices has greatly expanded the ability to collect such data continuously and in real-time. Despite these advancements, there remain significant challenges that impede the effective use and accuracy of the data collected by these devices. One of the primary challenges is the synchronization of data collected from multiple sensors attached to different parts of the body.

Traditionally, each wearable sensor timestamps its collected data using its internal clock. This approach assumes that each device's internal clock maintains a consistent and accurate time, which often is not the case. Over time, even extremely small discrepancies in clock accuracy can lead to a drift, resulting in significant misalignment of data streams when they are collated for analysis. Such misalignments can severely affect the interpretation and reliability of the health metrics being monitored.

Furthermore, the current state of the art typically requires extensive post-processing of the data to align these time-stamped data streams from each sensor module. This offline analysis is not only labor-intensive but also introduces the potential for human error, which can result in inaccurate assessments of health conditions. This is particularly problematic in environments where real-time data synchronization is crucial, such as in the monitoring of conditions that require the precise coordination of sensor readings to determine a health event or status.

Moreover, with the increasing number of sensors being used to monitor various health metrics, the complexity of data synchronization grows exponentially. This complexity can lead to increased time and resources spent on post-collection data adjustments. The inability to ensure real-time data integrity can result in delays in decision-making and can limit the potential for immediate intervention based on the analysis of the health-related data collected.

In a multi-sensor environment, for example where real-time monitoring and immediate feedback are important, there exists a pressing need for a solution that can obviate at least of limitations and/or drawbacks mentioned above.

### SUMMARY OF THE INVENTION

It is an object of the invention to provide for a method and a system that obviates at least one of the above mentioned drawbacks.

Additionally or alternatively, it is an object of the invention to provide for a modular system with wearable devices for collecting health-related data in an improved manner.

Additionally or alternatively, it is an object of the invention to provide for an improved method for collecting health-related data using a modular system with a hub and wearable devices.

Additionally or alternatively, it is an object of the invention to provide for a modular system with a hub and modular wearable devices connectable to the hub, that can more accurately collect health-related data.

Thereto, the invention provides for a modular system comprising a hub and modular wearable devices wirelessly connectable to the hub, wherein said wearable devices are attachable to parts of a body for collecting health-related data, wherein the wearable devices are allowed to collect data independent of each other, and wherein, when time-synchronized data is required, the hub is configured to send a trigger signal to the wearable devices for initiating and synchronizing measurements.

The modular system includes a hub and various modular wearable devices capable of wirelessly connecting to the hub and attaching to different body parts. The wearable devices are designed to independently collect data, which means that each device can function autonomously without relying on the operation of other devices. This independence in data collection allows for greater flexibility and reliability, as the failure or malfunction of one device does not impede the functionality of others. Moreover, the hub is specifically configured to enable synchronized measurements when required, by sending trigger signals to the wearable devices. This configuration facilitates the acquisition of time-synchronized data, which is important for applications requiring precise temporal correlation of health metrics, thereby ensuring high accuracy and consistency in health monitoring.

The hub, as the central component of the modular system, includes a configuration that serves an important function in the orchestration of time-synchronized data acquisition. Its ability to send precise trigger signals to the wearable devices addresses the need for temporal alignment in health-related data collection. When applications demand a strict correlation between the timestamps of data collected from different sensors or body parts, the hub provides significant advantages.

This triggering mechanism allows for the synchronized initiation of data collection across various wearable devices. By doing so, the hub ensures that the data from disparate sources is not only time-stamped but also contemporaneously gathered, preserving the temporal relationship between different physiological events or metrics. For example, in the measurement of Pulse Arrival Time (PAT) or Pulse Transit Time (PTT), which are important in assessing cardiovascular health, simultaneous data capture from multiple devices is important.

The hub achieves this effect by leveraging a master clock or similar timing control system that can emit synchronization pulses or signals with precision. In some examples, when these signals are received by the wearable devices, they initiate measurement at the exact same moment, effectively "locking" their internal clocks to the timing of the hub. This ensures that all data is collected within the same narrow time window, which is necessary for accurate cross-comparison and analysis of health metrics that change rapidly over time, such as heart rate or blood flow.

Moreover, the synchronization feature provided by the hub can significantly reduce temporal discrepancies that can otherwise arise from independent operation of the wearable devices. By effectively aligning the timing of data collection, the hub enhances the reliability of the data, which in turn increases the accuracy of subsequent health assessments and diagnoses.

Each wearable device may operate autonomously in its data collection capacity, however, the hub exerts an important role in synchronizing the devices when concurrent data acquisition is mandated. In some examples, the hub may employ various wireless communication protocols such as Bluetooth LE, WiFi, Zigbee, or other radio frequency communications to facilitate this functionality. Furthermore, in some examples, the modular nature of the system may extend to incorporate environmental sensors that, although not worn on the body, can provide contextual health-related data such as air quality, UV radiation levels, etc., that may influence the user's health parameters.

Optionally, the hub is configured to provide clock stimuli to the wearable devices for time synchronization of collected data.

Improved data synchronization accuracy can be obtained by equipping the hub with the capability to provide clock stimuli to the wearable devices. This ensures that the data collected is time-synchronized, which is important for accurate analysis and correlation of some health-related metrics. The clock stimuli facilitate the alignment of data collection timestamps across all wearable devices, thus enabling precise time-based comparisons and integrations of the health data. By utilizing a synchronization protocol that governs the data collection process, the reliability and validity of the health data acquired can be enhanced.

In some examples, the hub is configured to emit clock stimuli to the various wearable devices within the (modular) network. This significantly elevates the precision of the system: the enhancement of data synchronization accuracy. The provision of clock stimuli is not merely for coordination but is a critical requirement for maintaining the integrity of the time-based data collected from multiple sources on the body.

The transmission of these clock stimuli can be orchestrated through a synchronization protocol, a set of rules or an algorithm that the hub follows to ensure that each wearable device in the system aligns its internal clock to a master reference time. Consequently, each data point collected by the wearable devices carries a timestamp that is in strict accordance with the hub's master clock. This precision in timestamping is important for the accurate analysis and correlation of health-related metrics, particularly when dealing with physiological events that occur in different locations of the body and need to be temporally correlated with one another to provide meaningful insights.

For example, the synchronized measurement of electrical signals from an electrocardiogram (ECG) sensor and optical signals from a photoplethysmogram (PPG) sensor can reveal vital information about the cardiovascular system, such as the time interval between the onset of a heartbeat and the resulting pulse wave reaching a peripheral site. Any deviation in synchronization can lead to incorrect calculations of parameters like Pulse Transit Time (PTT), which can, in turn, lead to misinterpretation of cardiac health. By aligning the data collection timestamps through the dissemination of clock stimuli, the system allows for precise time-based comparisons and integrations of health data. This synchronized timestamping across some or all connected wearable devices enables the system to function as a coherent whole rather than as a collection of independent and discordant parts. This synchronization ensures that the data, when analyzed, reflects a true and accurate physiological state of the individual, thereby greatly enhancing the reliability and validity of the health data acquired. The robust and accurate data collected holds significant implications for patient monitoring and/or overall health management, allowing for informed medical decision-making based on precise and reliable health metrics.

Optionally, the hub is operable in a receive mode, for receiving data from the wearable devices, and in a transmit mode, for transmitting trigger signals to the wearable devices to initiate time synchronized data collection.

The modular system can feature a hub that operates in both a receive mode and a transmit mode. This dual-mode operation can provide a dynamic and efficient management of data transmission. In the receive mode, the hub effectively gathers data from the wearable devices, while in the transmit mode, it can actively dispatch trigger signals to initiate synchronized data collection. This bi-modal functionality enables the hub to control and coordinate the data flow and synchronization process seamlessly, thereby achieving real-time, synchronized data capture which is important for the prompt and accurate assessment of health conditions.

In receive mode, the hub may act as a central repository, effectively aggregating data transmitted by each of the wearable devices. This data collection is continuous and allows for a comprehensive stream of health-related metrics to be captured and stored for analysis. In some examples, the receive mode is important for the passive aspect of the system's operation, where the hub's primary role is to collect and log data without actively influencing the timing of the wearable devices' measurements. When the hub switches to transmit mode, it may adopt a more proactive role. In this state, the hub actively dispatches trigger signals to the wearable devices. These signals serve as a synchronization beacon, directing all wearable devices to commence their data collection simultaneously. This functionality is particularly important when the system needs to capture time-critical health events, where the simultaneous recording of physiological signals from multiple sensors is important for accurate diagnosis.

Advantageously, a seamless coordination of data flows can be obtained, allowing for an optimized balance between continuous monitoring and event-driven synchronized data capture. With real-time data synchronization, the system can ensure that the temporal relationships between different physiological signals are maintained, which is vital for the prompt and accurate assessment of conditions.

The dynamic switching between receive and transmit modes allows the system to conserve energy by synchronizing devices only as needed, rather than continuously, which would demand more power and can reduce the wearable devices' operational longevity. This intelligent management of power and data synchronization duties enhances the overall efficiency and effectiveness of the system, providing reliable real-time health monitoring important for both routine and critical care scenarios.

In some examples, the hub may switch between modes based on adaptive algorithms that optimize power consumption and data fidelity according to the specific needs of the user.

In some examples, the device can operate in both modes, namely the receive and transmit modes, concurrently.

In some examples, the hub can trigger devices using sub-millisecond precision for high-resolution data synchronization necessary in critical monitoring scenarios.

Optionally, time-synchronized data is required for computing at least one of a Pulse Arrival Time (PAT) or Pulse Transit Time (PTT) using at least two wearable devices.

The computation of critical cardiovascular metrics such as Pulse Arrival Time (PAT) or Pulse Transit Time (PTT) in an accurate way, are enabled by the system. This is made possible by the precise time synchronization of data captured by at least two wearable devices, which is a requisite for calculating these time-dependent measurements.

These metrics are important for evaluating the cardiovascular health of a user and can provide invaluable insights for both clinical diagnosis and personal health monitoring. The calculation of PAT and PTT relies on the precise temporal correlation of pulse signals captured from different locations on the user's body. This necessitates that data from at least two wearable devices be accurately time-synchronized. PAT refers to the time it takes for the blood pressure pulse wave to travel between two arterial sites, while PTT is the time it takes for the pulse wave to travel from the left ventricle of the heart to a peripheral site on the body. Both measurements are dependent on the velocity of the pulse wave, which in turn can be indicative of arterial stiffness, blood pressure, and other cardiovascular conditions.

The system has the capability to achieve this time synchronization, thereby allowing for the precise computation of PAT and PTT. This is accomplished through the coordinated triggering of data collection by the hub, which ensures that the wearable devices begin measuring at precisely the same moment in time. Without such synchronization, the collected data may reflect time lags introduced by asynchronous measurement starts, which would be detrimental and/or undermine the accuracy of PAT and PTT calculations and can lead to incorrect assessments of cardiovascular health.

By maintaining the necessary temporal alignment of cardiovascular signals, the system enables advanced cardiovascular monitoring. The ability to collect and correlate temporally aligned cardiovascular data is invaluable, as it can lead to early detection of cardiovascular anomalies, allow for the monitoring of changes over time, and facilitate the assessment of intervention outcomes.

Within this modular system, the requirement for time-synchronized data becomes especially salient when calculating metrics such as Pulse Arrival Time (PAT) or Pulse Transit Time (PTT), which necessitate coordinated measurements from at least two wearable devices. For example, one can measure the PAT by synchronizing a wearable device on the wrist with another on the chest or torso of the body. In alternate examples, additional biometric parameters, such as the synchronization of body temperature gradients or interstitial glucose levels, can also be calculated using similar synchronized time-stamping techniques, provided by the hub, to deduce circadian patterns or metabolic states.

Optionally, time synchronization of data from the wearable devices is achieved through the hub using a master clock that is configured to provide one or more synchronization pulses to the wearable devices.

A master clock within the hub can be used to achieve synchronization of data from wearable devices. This master clock can provide synchronization pulses to the wearable devices, ensuring that all devices are operating on a common time frame. A precise alignment of data collection instances across all devices can be obtained, which is particularly important for the analysis of time-sensitive health metrics. This centralized synchronization method simplifies the complexity of managing multiple time sources and ensures the integrity and accuracy of the synchronized data.

The master clock within the hub can serve as the cornerstone for data synchronization across all wearable devices. This master clock can be used to dispatch synchronization pulses to the wearable devices, unifying their operation within a common time frame. It can effectively ensure that the time at which data is collected from each device is precisely aligned with the others, in an advantageous way.

Advantageously, it allows for the accurate and simultaneous collection of data, which is indispensable for the analysis of health metrics that are sensitive to timing discrepancies. In instances where milliseconds can represent meaningful differences in physiological responses-such as in the measurement of heart rate variability or the conduction of neural signals-this precise alignment is important. Furthermore, by centralizing the time synchronization within the hub, the system simplifies the otherwise complex task of managing multiple independent time sources. Without a master clock, each wearable device would need to maintain its own time-keeping mechanism and the system would require a protocol to handle the inevitable variations between these time sources. This can introduce discrepancies in the data collection process, thereby complicating data analysis and compromising the data's integrity.

The centralized master clock approach also brings about an increase in the reliability of the system. It ensures that all wearable devices are synchronized to a single, accurate time source, thus preventing the accumulation of timing errors that can occur if each device relied on its own clock. This translates to a consistency in data collection over both short and long-term monitoring periods, which is critical for chronic health condition tracking, where trends and patterns over time are key to understanding and managing the condition.

Furthermore, the synchronization provided by the master clock enables more efficient processing and analysis of the collected data. Since the timestamps of the collected data are aligned, the need for post-collection data alignment and time correction is eliminated, streamlining the data analysis process.

Optionally, the hub performs pulsed synchronization of signals in real-time.

This provides the advantage of immediate and responsive data synchronization across wearable devices. This real-time capability ensures that data from different sensors is accurately time-stamped, enabling precise cross-comparison and analysis. A highly accurate temporal dataset can be achieved, which can be vital for the timely detection and monitoring of health events or conditions. This pulsed synchronization is important for applications that require data to be collected and analyzed in a synchronous manner, without delay or temporal discrepancies.

This level of responsiveness can be particularly important when dealing with health-related metrics that are subject to rapid changes and require immediate capture and analysis to be meaningful. Real-time pulsed synchronization can create a highly accurate and temporally cohesive dataset. Each piece of health-related data collected from the various sensors can be precisely time-stamped at the moment of acquisition. This temporal precision can be indispensable for the cross-comparison of data points and thorough analysis. For instance, when assessing the cardiovascular function of a user, the concurrent capture of an electrocardiogram (ECG) trace and a photoplethysmogram (PPG) waveform is important. Any delay or inconsistency in timing between these signals can significantly affect the calculated metrics, such as Pulse Transit Time (PTT), leading to inaccuracies in assessing the user's cardiovascular health.

Moreover, this pulsed synchronization methodology is integral to applications requiring that data from different sensors be collected and analyzed synchronously. In scenarios where split-second changes can denote critical health events, like arrhythmia detection or the onset of a seizure, the system's capacity to synchronize data collection in real-time is important. It ensures that such events are captured comprehensively, providing a robust basis for immediate clinical decision-making or triggering timely alerts for individuals monitoring their health conditions.

Optionally, the system is configured to provide a bi-directional wireless connectivity between the hub and the wearable devices, wherein the bi-directional wireless connectivity is also used for transmitting and receiving stimuli for synchronizing data collection in real-time.

A bi-directional wireless connectivity between the hub and the wearable devices, not only allows for efficient data transmission but also the transmission and reception of synchronization stimuli. This bi-directional communication ensures that devices can be updated or controlled in real-time, providing the technical effect of a streamlined and responsive synchronization process. Additionally, the real-time transfer of stimuli for synchronizing data collection enhances the system's capability to promptly adjust to the requirements of time-sensitive data acquisition, thereby increasing the system's overall efficacy in health monitoring.

Bi-directional wireless connectivity can be obtained by means of a dual-way communication channel that extends beyond mere data transfer. It may encompass the transmission and reception of synchronization stimuli, which are important for maintaining precise timing coordination among the various devices.

A dynamic interaction between the hub and wearable devices can be facilitated, allowing not just for the efficient and reliable transfer of health-related data to the hub but also for the conveyance of commands and synchronization signals back to the wearable devices. This capability can be important when adjustments to the devices' operation need to be made swiftly, such as changing the frequency of data capture or updating the devices' firmware in situ, without the need for physical interfacing.

Advantageously, the bi-directional communication can be used to obtain a streamlined and highly responsive synchronization process. In the context of health monitoring, where the timeliness of data acquisition can be important, the ability to send synchronization stimuli in real-time significantly enhances the system's capability to adapt to the requirements of time-sensitive data collection. This is not just about ensuring that the collected data is synchronized, but also about the capacity to react to live events, such as triggering an immediate measurement in response to a detected anomaly in the user's vital signs.

Moreover, this real-time transfer of stimuli for synchronizing data collection means that the system can readily align the measurement intervals of all connected devices to capture transient health events accurately. For example, if a patient's cardiac event requires immediate capture of ECG and blood pressure data simultaneously, the system can trigger an immediate synchronized measurement across these sensors, ensuring that the event is recorded with temporal fidelity.

Different two-way wireless connectivity mechanisms between the hub and the wearable devices can be employed. In some examples, the bi-directional connectivity may be secured by end-to-end encryption to preserve the integrity and confidentiality of the health-related data being synchronized and transmitted.

Optionally, the hub is usable for recharging devices.

The hub may thus not only be used for managing data and synchronization but also for recharging the wearable devices. This provides for a convenient and efficient way to maintain the wearable devices' power supply without needing separate charging mechanisms. This integration promotes user compliance and device readiness by ensuring that the wearable devices are charged and functional whenever required.

By providing a single point where users can charge their devices, the likelihood of devices being fully powered and ready for use when needed is significantly increased. This convenience ensures that users are more to adhere to recommended monitoring regimes, hence promoting continuous health monitoring.

Users are relieved of the burden of remembering to charge multiple devices individually, which can often lead to lapses in device readiness and consequent gaps in health data collection. Instead, they can maintain all wearable devices' power supply through one interaction with the hub. Hence, the opportunity for continuous, uninterrupted health monitoring can be improved. This contributes to the accumulation of a more complete and reliable health data set, which is important for long-term health management and analysis.

In some examples, the hub incorporates wireless charging capabilities, enabling the wearable devices to be recharged simply by placing them in proximity to the hub. In alternate examples, the hub may feature a docking system with pins for a more traditional charging connection.

In some examples, the wearable devices may incorporate energy-harvesting subsystems to charge devices when they are not in active use, thereby extending their operational periods. The energy-harvesting subsystems may for example operate based on movement/acceleration.

Optionally, the hub is configured to transmit data to a remote unit. In some examples, the hub is configured to send data to the cloud, for example by means of wired or wireless communication.

Optionally, the system comprises a configurational modularity, enabling the detachable connection of a plurality of wearable devices to the hub, each wearable device being configured to wirelessly interface with the hub for bi-directional communication and data transmission, wherein the wearable devices, upon connection to the hub, are operable to autonomously collect health-related data from designated body parts and transmit said data to the hub, further wherein the hub is equipped with synchronization circuitry operable to distribute synchronization signals to the wearable devices for orchestrated data collection and time-aligned data aggregation when time-synchronized measurements are needed.

The modular system can be provided with configurational modularity, which provides the advantage of scalable and customizable health monitoring. The system enables the detachable connection of various wearable devices to the hub, allowing for a tailored approach to health data collection. This customization permits the system to adapt to the specific health monitoring needs of different users. Additionally, the synchronization circuitry within the hub facilitates orchestrated data collection and time-aligned data aggregation when necessary, thereby enhancing the precision and reliability of health data analysis.

The modular system significantly enhances user adaptability through its configurational modularity. A wide range of wearable devices that can be selectively attached and detached from the hub can be supported by the system. This flexibility allows for a personalized health monitoring experience, as users can customize the system to monitor specific health metrics relevant to their individual needs.

This modularity enables the system to be dynamic and versatile, accommodating the inclusion or exclusion of different wearable devices as required. This means that the system can be easily adapted for different health conditions or monitoring objectives, without the need for acquiring a completely new set of equipment. Moreover, the synchronization circuitry within the hub can ensure time-aligned data aggregation from the connected wearable devices. When synchronized measurements are required/needed, the hub's circuitry can be responsible for sending out synchronization signals to all wearable devices, ensuring that data is collected in a coordinated fashion. This is particularly important for accurate health data analysis, as it allows for the comparison and integration of data from multiple sources, based on a common temporal framework. This orchestrated approach to data collection not only improves the precision of health data but also increases the reliability of the analysis drawn from this data. In scenarios where multiple physiological parameters need to be assessed in relation to one another, such as the interplay between heart rate and physical activity, the system's ability to synchronize data collection becomes of great importance.

In some examples, each modular device is configured for wireless interfacing with the hub, capable of autonomous operation for health data acquisition from assigned body locations. For instance, a module can be specifically designed for attachment to the user's lower back to monitor posture and spinal health.

In some examples, the wearable devices may also feature modular sensor units themselves, allowing users to further customize or upgrade individual sensors.

Optionally, the wearable devices are equipped with a different sensing mechanisms operable to collect different health-related metrics including cardiovascular data, respiratory data, posture data, and fluid accumulation data, wherein said sensing mechanisms are chosen from a group comprising at least one of: electrocardiogram sensor, photoplethysmogram sensor, heart rate and variability sensor, respiratory rate and/or depth sensor, accelerometer, and bioimpedance sensor, blood glucose level sensor, electrodermal activity sensor, electromyography sensor, electroencephalography sensor or biochemical sensor, and wherein the collected data is wirelessly transmissible to the hub for analysis and/or processing.

The capability to utilize a diverse array of sensing mechanisms within the wearable devices, enables the collection of a broad spectrum of health-related metrics. This variety ensures comprehensive health monitoring, from cardiovascular to biochemical parameters. The wireless transmissibility of collected data to the hub for analysis and/or processing allows delivering personalized and detailed health insights.

The system can capture a comprehensive array of health-related metrics, such as cardiovascular, respiratory, posture, and fluid accumulation data, among others. This range of sensing options, including but not limited to electrocardiogram sensors, photoplethysmogram sensors, accelerometers, and bioimpedance sensors, allows for the assessment of multiple facets of a user's health simultaneously. By incorporating such a wide range of sensors, a thorough and nuanced health assessment capability can be obtained. For instance, by analyzing data from an electrocardiogram sensor alongside a photoplethysmogram sensor, the system can provide insights into the user's heart function and peripheral blood flow, offering a comprehensive cardiovascular profile. The inclusion of sensors for detecting respiratory rate, posture, and even blood glucose levels means that the system can cater to various health monitoring needs, from fitness tracking to chronic condition management.

The capability of these wearable devices to wirelessly transmit the collected data to the hub enables the consolidation and processing of diverse health metrics in one central location, which not only facilitates the ease of data management but also enhances the potential for more sophisticated health analyses and insights. For instance, various algorithms (e.g. machine learning algorithms) can be employed in the hub to interpret the collective data, identify patterns, and even predict potential health issues.

The wireless transmission of data can support real-time or near-real-time monitoring and analysis, leading to timely interventions and responsive healthcare.

Various sensors may be used. Other examples include, a galvanic skin response sensor to measure stress levels, or a non-invasive blood pressure sensor to continuously monitor blood pressure, etc.

In some examples, the system can integrate with fabric-based sensors woven into and/or attached to clothing, providing a seamless and non-obtrusive health monitoring experience.

In some examples, the sensors may employ artificial intelligence or machine learning algorithms to adaptively select the appropriate sensor for data collection based on the current activity or health state of the user.

Optionally, the system includes at least an electrocardiogram, ECG, sensor, and a photoplethysmogram, PPG, sensor, wherein the hub is configured to orchestrate time synchronization between the ECG and PPG measurements of the ECG sensor and PPG sensor, respectively.

The system can be configured to facilitate time synchronization between ECG and PPG measurements. This synchronization is advantageous for correlating the electrical and optical readings of the heart's activity, important for detailed cardiovascular analysis. An enhanced accuracy in determining cardiac events and the health of the cardiovascular system can be obtained, which can be achieved through the precise temporal alignment of ECG and PPG data. This ability to coordinate complex sensor data is important for advanced health diagnostics and monitoring.

ECGs record the electrical activity of the heart and are important for monitoring heart rhythm and identifying arrhythmias, while PPGs use light to measure changes in blood volume in the skin, thereby providing information on blood flow and can be used to deduce heart rate and blood oxygen saturation. The time-synchronized ECG and PPG measurements allow for a more nuanced interpretation of cardiovascular events. By aligning these two types of data in time, the time interval between the electrical initiation of a heartbeat (as shown by the ECG) and the resulting pulse at the skin level (as shown by the PPG) can be effectively assessed. This interval is referred to as Pulse Transit Time (PTT), which can be a proxy for arterial stiffness and blood pressure, among other cardiovascular insights.

By providing a clearer temporal relationship between the electrical and optical readings of heart activity, more informed diagnoses regarding the cardiovascular system's status can be made. Furthermore, synchronized data can help detect anomalies that may be indicative of conditions such as arterial blockages or irregular heartbeats. Furthermore, the system can give users a more comprehensive view of their heart's health, incorporating both electrical activity and blood flow data. In some examples, a continuous, non-invasive monitoring of blood pressure can be possible through the analysis of PTT, which relies heavily on the precise timing of ECG and PPG signals.

In some examples, the ECG sensor can be integrated into a smart clothing while the PPG sensor can be located in a smart ring or bracelet. In other examples, the ECG and PPG sensors may utilize skin-adhesive technologies that allow them to be discreetly affixed to the skin, thus providing continuous and unobtrusive monitoring.

In some examples, the ECG and PPG sensor are integrated into one sensor device.

In some examples, the system with the ECG and PPG sensor also has a bioimpedance sensor and an accelerometer. The addition of these two parameters can enhance the measurements. For example, the accelerometer may identify, based on the movement of the user, whether the timing for initiating a measurement is adequate.

Optionally, the ECG sensor is configured to be positioned on a chest, and the optical PPG sensor is configured to be positioned on another part of the body.

This arrangement can allow for the optimal capture of electrical signals directly from the heart and peripheral blood flow indications, respectively. This sensor placement can improve accuracy and reliability of cardiovascular measurements, important for detecting and analyzing heart-related health conditions.

The ECG sensor can be positioned on or near the chest, where it can best capture the heart's electrical signals. This placement can provide for a clearer and more accurate recording of electrical impulses. On the other hand, the PPG sensor, which detects blood volume changes by emitting and capturing light through the skin, can be placed on a different part of the body, such as the wrist, finger, or earlobe. These areas are chosen because they are rich in capillaries and thus provide a clear signal of blood flow that is affected by the pumping action of the heart.

With the ECG and PPG sensors placed at optimal locations, the time difference between the heart's electrical activity and the observed pulse wave at the peripheral site can be accurately measured. This enables precise calculation of Pulse Transit Time (PTT), which has important clinical implications for assessing vascular health.

According to an aspect, the invention provides for a hub configured to wirelessly connect to a plurality of modular wearable devices, each wearable device being attachable to a part of a body for collecting health-related data, wherein said hub comprises a communication module for facilitating independent data collection by each of the wearable devices, and a synchronization module for sending a trigger signal to the wearable devices to initiate and synchronize measurements when time-synchronized data is required, said synchronization module being activated based on predefined conditions or user input.

The hub can wirelessly connect to various wearable devices, with a communication module that supports independent data collection and a synchronization module for coordinated measurements. The hub can manage a diverse range of data collection tasks simultaneously while maintaining the option for synchronized data capture as needed. A versatile hub can be obtained that serves as the central command for both the autonomous operation of individual wearable devices and the integrated synchronization of data. This flexibility can address varying health monitoring demands, ensuring that data is gathered both continuously and in concert when temporal correlation is necessary. The synchronization module's activation, which can be based on predefined conditions or user input, adds a layer of user control, enabling the system to adapt to specific monitoring requirements in real time, thereby enhancing the personalization of health management strategies.

The system may allow wearable devices to operate independently, gathering data autonomously without the need for synchronization. This means that each device can function on its own to monitor specific health metrics and store or send this data back to the hub. This is useful for continuous monitoring where immediate temporal correlation with data from other devices is not necessary.

Furthermore, when required, the hub can activate its synchronization module to ensure that data collection is perfectly timed across devices. This coordinated synchronization is important for measurements where the temporal relationship between different data points is critical, such as the coordination between heart rate data from an ECG and activity data from an accelerometer during an exercise stress test.

The hub's ability to support both independent and synchronized data collection makes it highly versatile. It can adapt to various scenarios, from long-term general health monitoring to specific diagnostic tests that require data integration from multiple sources.

The hub can effectively act as a central command center, orchestrating the activities of all connected wearable devices. It can ensure that a vast array of health data is collected efficiently and effectively.

With the hub's capacity for both autonomous and coordinated operation, it can meet diverse health monitoring needs. It allows for a personalized approach to health management, where the type of data collection can be adjusted based on the user's requirements.

The activation of the synchronization module based on predefined conditions or user input indicates that the system can be dynamically adapted. If a health event is detected that requires synchronized data for accurate assessment, the hub can respond instantly, ensuring that critical data is not missed.

In some examples, the synchronization module may activate upon detecting certain physiological thresholds or after a manual prompt from a user, e.g. through a connected app interface, voice command, etc.

In some examples, the hub may use adaptive triggering, where it learns the optimal times to synchronize devices based on past data patterns or predictive modeling, thus reducing the need for manual inputs or pre-set conditions.

According to an aspect, the invention provides for a method of configuring a modular system for collecting health-related data, wherein the method includes providing a hub and modular wearable devices wirelessly connectable to the hub, wherein said wearable devices are attachable to parts of a body for collecting health-related data, wherein the wearable devices are allowed to collect data independent of each other, and wherein, when time-synchronized data is required, the hub is configured to send a trigger signal to the wearable devices for initiating and synchronizing measurements.

A tailored setup process can be obtained that adapts to various health monitoring requirements. By providing a hub and wearable devices that can be wirelessly connected and attached to body parts as needed, the system allows for individualized/personalized health tracking. A customizable, user-specific health monitoring regimen can be obtained where the initiation and synchronization of measurements by the hub can be configured to occur only when time-synchronized data collection is required. This selective synchronization conserves system resources and enhances the efficiency of the system, ensuring that enhanced data accuracy is available on-demand, thereby improving the quality of health assessments.

The sensors can be chosen and placed according to what health metrics need to be monitored, such as heart rate, blood oxygen levels, physical activity, etc. Instead of continuously collecting data from all sensors, which can be resource-intensive, the hub can be set up to initiate and synchronize measurements only when necessary. This selective approach to data collection can extend the battery life of the devices, reduce data storage requirements, and streamline the analysis process.

When the system does synchronize devices, it is designed to do so with precision. This ensures that the collected data is accurate and reliable, which is particularly important for health assessments that rely on the temporal correlation of data from different sources.

According to an aspect, the invention provides for a method for collecting health-related data using a modular system, the method comprising: providing a hub; providing a plurality of modular wearable devices wirelessly connectable to the hub, wherein said wearable devices are attachable to parts of a body; allowing the wearable devices to collect health-related data independent of each other; determining whether time-synchronized data collection is required, and in response to determining that time-synchronized data collection is required, sending a trigger signal from the hub to the wearable devices for initiating and synchronizing measurements; collecting the health-related data from the wearable devices; and transmitting the collected health-related data to the hub for further processing and/or analysis.

The method offers the advantage of a structured and efficient process for both autonomous and synchronized data collection, allowing for the wearable devices to independently gather data but also provides for the capability to initiate and synchronize measurements through the hub when necessary. A highly adaptable data collection process that ensures comprehensive health monitoring by capturing both continuous and event-specific health data can be obtained. By determining when time-synchronized data collection is required and initiating this process via the hub, the method can effectively ensure that the collected health-related data is both comprehensive and precise, facilitating detailed analysis and enhancing the potential for early detection and intervention in health-related matters.

Each wearable device can function independently to collect data continuously or at intervals set by user preference or by a healthcare provider's recommendation, for example. This autonomy ensures that a baseline of health-related data is always being recorded, which can be important for tracking trends over time. Furthermore, the hub can initiate synchronized data collection across some or all wearable devices when needed. This is important for certain health events or conditions where the temporal relationship between different health metrics is critical.

In some examples, by not requiring all devices to be synchronized at all times, the system can conserve energy and computational resources, enhancing the battery life and performance of the wearable devices.

The advantageous synchronization mechanism means that data from different devices can be accurately aligned in time.

It will be appreciated that any of the aspects, features and options described in view of the modular system apply equally to the methods and the described devices and hub. It will also be clear that any one or more of the above aspects, features and options can be combined.

### BRIEF DESCRIPTION OF THE DRAWING

The invention will further be elucidated on the basis of exemplary embodiments which are represented in a drawing. The exemplary embodiments are given by way of non-limitative illustration. It is noted that the figures are only schematic representations of embodiments of the invention that are given by way of non-limiting example.

In the drawing:
Fig. 1 shows a schematic diagram of an embodiment of a system;
Fig. 2 shows a schematic diagram of an embodiment of a system;
Fig. 3 shows a schematic diagram of an embodiment of a system; and
Fig. 4 shows a schematic diagram of a method.

### DETAILED DESCRIPTION

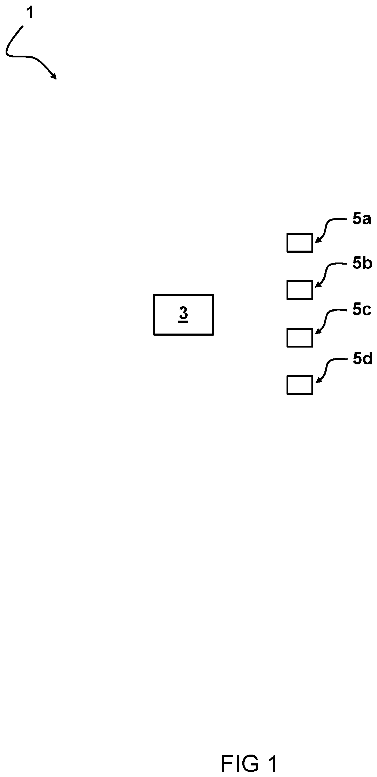

Fig. 1 shows a schematic diagram of an embodiment of a modular system 1. The system 1 includes a hub 3 and modular wearable devices 5a-d wirelessly connectable to the hub 3. The wearable devices 5a-d are attachable to parts of a body for collecting health-related data. The wearable devices 5a-d are allowed to collect data independent of each other. Furthermore, when time-synchronized data is required, the hub 3 is configured to send a trigger signal to at least a subset of the wearable devices 5a-d for initiating and synchronizing measurements.

The hub 3 may be used for time synchronization of signals from master and slave devices. The hub 3 is employed for triggering measurement instances and sending external stimuli to both master and slave devices 5a-d for time synchronization of signals. Hence, an advantageous pulsed/triggered synchronization of signals can be obtained in real-time. The hub 3 may have an independent clock as a common unit for providing synchronization pulse to devices 5a-d.

By employing a pulsed synchronization approach, the system 1 can mitigate the risk of temporal discrepancies that can arise from less responsive and/or less accurate synchronization methods.

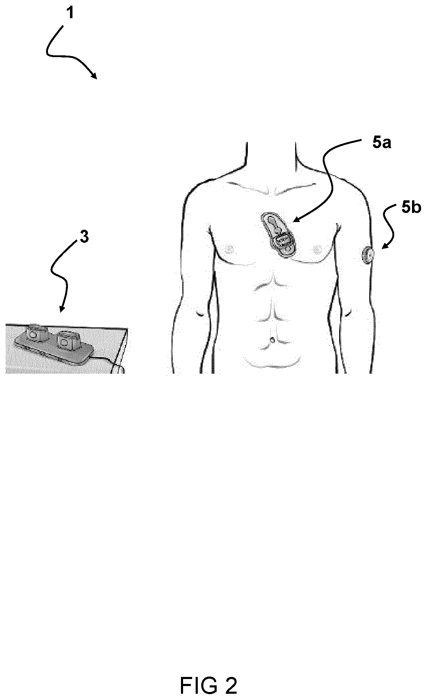

Fig. 2 shows a schematic diagram of an embodiment of a system 1. In this example, the system 1 may have a multi-modal distributed group of sensors with a common communication hub 3. In this example, two sensors 5a, 5b are shown, but the system may have a different number of sensors.

In some examples, the system 1 has modular devices for heart failure management. For instance, electrophysiological parameters can be measured from different regions of the body depending on the nature of the parameter to be measured. For instance, ECG from the chest/torso, skin oxygenation from the arm and fingers, EEG from the head, electro-oculography in the forehead, electromyography (EMG) from the arms and/or other muscles, bioimpedance from the chest, etc. Location of a particular sensor for measurement can be determined by several factors such as the region of interest, signal quality, type of signals measured among others. Furthermore, depending on the clinical condition of the patient, not all parameters may be required to be measured during their care cycle. Advantageously, the system 1 employs a modular approach for wearable devices for data collection. Depending on patient and measurement requirements, a particular set of wearable devices or sensors can be deployed for measuring vital signs and can be wirelessly connected to a common hub 3 for efficient data transfer.

In some examples, a combination of multiple wearable devices can be connected wirelessly to a common communication hub 3. In the shown example, a chest patch 5a is utilized for measuring vital signs such as the ECG, heart rate, respiratory rate, and a bioimpedance sensor for measuring fluid accumulation in the lungs. Furthermore, a patch 5b on the arm for measuring photoplethysmography (PPG) signals is used. Additionally or alternatively, various other sensors may be used, such as a bioimpedance device on the leg for measuring fluid accumulation in the peripheral parts of the body.

The wearable devices may be battery powered for long-term measurement and the devices can be wirelessly connected through Bluetooth to a common communication hub for data transfer (bi-directional). Various other communication protocols may be used.

In this example, the hub also acts as a charging station to re-charge the devices. Furthermore, the hub may be connectable to a cloud data warehouse for transferring data from the wearables devices to a cloud repository. The processed information from the cloud can be displayed in dashboards and for patient centric mobile applications.

In some examples, the system 1 may have a chest worn master device with sensors for measuring ECG, Heart Rate and Variability, Respiratory Rate and Depth, Accelerometer for measuring the posture of the patient and bioimpedance sensors for measuring fluid accumulation in the lungs. One or more secondary / slave devices may be attached on the peripheral parts of the body such as the arm or the legs. The secondary devices include PPG sensors for measuring volumetric variations in blood circulation, a 3-axis accelerometer for measuring the peripheral arm / leg position. An external hub can be provided that is used for triggering measurement instances and sending external stimuli to both master and slave devices for time synchronization of signals. The external hub can also be used for recharging master and slave devices and a central hub for downloading data wirelessly from the devices. For example, the external hub may include cellular connectivity for uploading data to a cloud data warehouse.

In some examples, the external hub 3 may include both Bluetooth low energy (BLE) receive mode for receiving vital sign data from the wearable devices and can operate in transmit mode for providing trigger signals to the master and slave wearable devices to initiate time synchronized data collection. Master and slave devices may be configured to continue to collect data independent of each other. When time synchronized data is required for computing Pulse Arrival Time (PAT) or Pulse Transit Time (PTT), a trigger signal may be sent from the external hub to the master and slave device for initiating and synchronizing signals.

The system 1 may include an independent external hub used for time synchronization of signals from master and slave devices. The bi-directional connectivity in the Master, slave and the Hub can be used for transmitting and receiving external stimuli to synchronize data in real-time. The external hub can be multi-functional and can be used for recharging the devices, download / upload of data from the devices to the cloud and for providing external clock stimuli to the master and slave device.

The device can be deployed in modular fashion based on application requirements and care pathway requirements. Master and slave combination of devices can be selected based on the clinical conditions (i.e. early onset, critical care, post-operative care), clinical dependency or can be deployed during certain time of the day (e.g. chest measurement during the day and PTT measurement during the night), i.e. time-dependent.

Time synchronization of data from independent sensor modules can be a complex challenge because of the required accuracy in measurement. Time synchronization usually involves independent data stream from two or more devices that are time stamped and correlated in offline analysis. An external hub 3 with an independent clock may be used in a common control unit for providing synchronization pulse to both master and slave devices. Pulsed synchronization of signals in real-time by communication between the hub, master and slave devices provides significant advantages as elucidated in this disclosure.

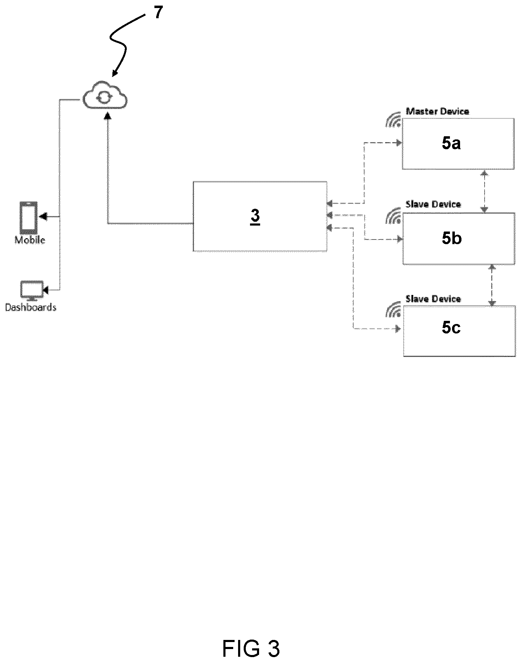

Fig. 3 shows a schematic diagram of an embodiment of a system 1. In this example, the hub 3 is connectable to a cloud system 7. This connection may be wireless or wired. Data from the cloud system 7 may be accessed by digital dashboards, mobile phones, computers, etc. The hub 3 may be wirelessly connectable to the plurality of modular devices 5a, 5b, 5c. In this example, the first sensory device 5a is a master device. Furthermore, two slave sensory devices 5b, 5c are provided. These devices may for instance be further operatively controlled at least partially by the master device. The modular devices 5a-c may be communicatively coupled to each other. In some examples, the master device 5a can communicate with the slave devices. However, it is also envisaged, in some examples, that slaves may communicate with each other.

In some examples, the external hub 3 has a dual-mode functionality. For instance, the external hub 3 may be configured to possess the capability to both receive and transmit data via Bluetooth Low Energy (BLE), a power-efficient variant of the classic Bluetooth wireless communication technology. In receive mode, the hub may collect health-related data from the wearable devices. These wearables may include sensors for monitoring parameters such as heart rate, temperature, blood oxygen levels, etc. In transmit mode, the hub may send out 'trigger signals' to the wearable devices. These signals can instruct the devices when to start collecting data such that all the devices begin recording simultaneously or at specific relative time intervals.

In distributed systems, the terms "master" and "slave" are often used to describe the relationship between two devices. Typically, the master device takes the lead in initiating communication or has control over other devices in the system. This salve device typically waits for instructions from the master device or another control entity (cf. hub 3).

Both the master and slave devices, in this system 1, can operate independently, which means they can continue their data collection without relying on each other. However, for certain measurements, such as Pulse Arrival Time (PAT) or Pulse Transit Time (PTT) that require data from both devices to be perfectly aligned, synchronization may be employed in an advantageous way. This can be achieved with the hub 3 operating in transmit mode.

In some examples, the master, slave, and hub are all equipped with BLE communication features that allow them to send and receive data. This exemplary bi-directional communication protocol can enable real-time synchronization. Other communication protocols may also be used.

In some examples, the system 1 employs an external hub 3, equipped with its independent clock, that acts as the central control unit. Instead of aligning data after collection (offline analysis), the hub 3 sends synchronization pulses in real-time. Upon receiving these pulses, both the master and slave devices timestamp their data. This process ensures that when the data is later analyzed, all readings from the different devices can be perfectly aligned.

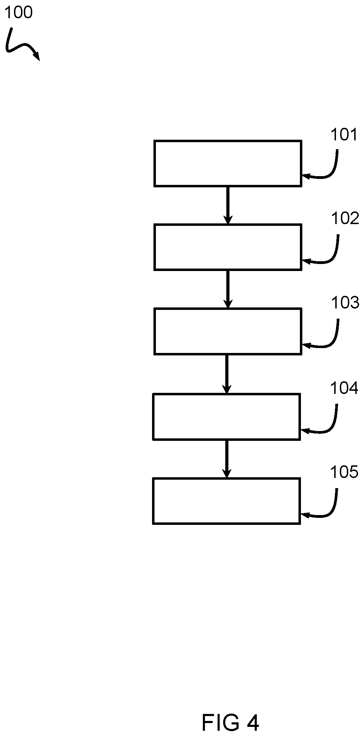

Fig. 4 shows a schematic diagram of a method 100 for collecting health-related data using a modular system. In a first step 101, a hub and a plurality of modular wearable devices wirelessly connectable to the hub are provided, wherein said wearable devices are attachable to parts of a body. In a second step 102, the wearable devices are allowed to collect health-related data independent of each other. In a third step 103, it is determined whether time-synchronized data collection is required, and in response to determining that time-synchronized data collection is required, a trigger signal from the hub to the wearable devices for initiating and synchronizing measurements is sent. In a fourth step 104, the health-related data from the wearable devices is collected. In a fifth step 105, the collected health-related data is transmitted to the hub for further processing and/or analysis.

Time synchronization can ensure that data from different sensors is collected and analyzed in a coordinated and aligned manner. This is of great importance, especially when measurements from separate sensors have to be compared or analyzed together to extract meaningful information.

Time synchronization demands precision. Even slight offsets in timestamps can lead to misleading results, especially when physiological signals with small time differences are under scrutiny.

Each sensor may generate its own stream of data. When multiple sensors are involved, there's a need to align these streams so that data points correspond to the same timeframes. This alignment can be ensured without the data streams interfering with each other.

The clock of the external hub may ensure a consistent and standardized time source, away from the potential variances of each sensor's internal clock.

In a synchronization setup, devices can be categorized as masters or slaves. A master device often takes a lead role in setting the synchronization standard, while slave devices adjust their operations to match this standard. In some examples, both master and slave devices rely on the external hub for synchronization.

The pulsed synchronization may provide for a real-time approach to synchronization. Instead of relying on post-hoc adjustments, the hub sends synchronization signals or "pulses" at (regular) intervals. Devices (both master and slave) can receive these pulses and adjust their clocks or timestamp their data accordingly. As the devices adjust in real-time to the pulses, there's minimal drift, ensuring better-aligned data streams. Also, by doing this in real-time, the need for extensive offline corrections is reduced.

In some examples, the sensors are configured to be capable of receiving these synchronization pulses and reacting in real-time.

In some examples, the hub has communication modules, often employing technologies like Bluetooth, Wi-Fi, or custom radio frequencies, enabling it to transmit synchronization pulses and receive data from sensors.

In some examples, upon startup or at predetermined intervals, devices connect to the hub and establish their role. While the hub remains the ultimate authority on time, the master device may play an intermediary role in relaying synchronization pulses to slave devices or directing traffic.

In some examples, slave devices may send feedback to the hub, detailing any observed drift or time discrepancy. This loop allows the hub to make real-time adjustments, further enhancing synchronization precision.

In some examples, the hub's independent clock is utilized to create synchronization pulses at regular intervals. These pulses may be signals with precise timestamps indicating the 'true' time as per the hub's clock.

In some examples, these pulses are then transmitted to at least a subset of connected devices. In some cases, the pulses may be transmitted to all connected devices.

In some examples, upon receiving a pulse, each receiving sensor device may captures the pulse's timestamp, compares it with its internal timestamp, adjusts its internal clock to align with the received timestamp, and correcting any potential drift. Given that communication isn't instantaneous, the hub and sensors may account for transmission latency. In some examples, the system (e.g. hub) can measure and compensate for this latency, ensuring that synchronization remains precise.

In some examples, if a device misses a synchronization pulse (due to interference or other issues), error-handling protocols may be used to ensure that the device either requests another pulse or waits for the next scheduled pulse to resynchronize.

In some examples, as sensors collect data, they timestamp each data point based on their now-synchronized internal clocks. This can ensure that data from different sensors aligns temporally.

In some examples, sensors periodically send their data to the hub. This data, already synchronized, can be aggregated, analyzed, or relayed to other systems by the hub.

It will be appreciated that the method may include computer implemented steps. All above mentioned steps can be computer implemented steps. Embodiments may comprise computer apparatus, wherein processes performed in computer apparatus. The invention also extends to computer programs, particularly computer programs on or in a carrier, adapted for putting the invention into practice. The program may be in the form of source or object code or in any other form suitable for use in the implementation of the processes according to the invention. The carrier may be any entity or device capable of carrying the program. For example, the carrier may comprise a storage medium, such as a ROM, for example a semiconductor ROM or hard disk. Further, the carrier may be a transmissible carrier such as an electrical or optical signal which may be conveyed via electrical or optical cable or by radio or other means, e.g. via the internet or cloud.

Some embodiments may be implemented, for example, using a machine or tangible computer-readable medium or article which may store an instruction or a set of instructions that, if executed by a machine, may cause the machine to perform a method and/or operations in accordance with the embodiments.

Various embodiments may be implemented using hardware elements, software elements, or a combination of both. Examples of hardware elements may include processors, microprocessors, circuits, application specific integrated circuits (ASIC), programmable logic devices (PLD), digital signal processors (DSP), field programmable gate array (FPGA), logic gates, registers, semiconductor device, microchips including Bluetooth controllers (BLE), chip sets, et cetera. Examples of software may include software components, programs, applications, computer programs, application programs, system programs, machine programs, operating system software, mobile apps, middleware, firmware, software modules, routines, subroutines, functions, computer implemented methods, procedures, software interfaces, application program interfaces (API), methods, instruction sets, computing code, computer code, et cetera.

Herein, the invention is described with reference to specific examples of embodiments of the invention. It will, however, be evident that various modifications, variations, alternatives and changes may be made therein, without departing from the essence of the invention. For the purpose of clarity and a concise description features are described herein as part of the same or separate embodiments, however, alternative embodiments having combinations of all or some of the features described in these separate embodiments are also envisaged and understood to fall within the framework of the invention as outlined by the claims. The specifications, figures and examples are, accordingly, to be regarded in an illustrative sense rather than in a restrictive sense. The invention is intended to embrace all alternatives, modifications and variations which fall within the scope of the appended claims. Further, many of the elements that are described are functional entities that may be implemented as discrete or distributed components or in conjunction with other components, in any suitable combination and location.

In the claims, any reference signs placed between parentheses shall not be construed as limiting the claim. The word 'comprising' does not exclude the presence of other features or steps than those listed in a claim. Furthermore, the words 'a' and 'an' shall not be construed as limited to 'only one', but instead are used to mean `at least one', and do not exclude a plurality. The term "and/or" includes any and all combinations of one or more of the associated listed items. The mere fact that certain measures are recited in mutually different claims does not indicate that a combination of these measures cannot be used to an advantage.

## Claims

1. A modular system comprising a hub and modular wearable devices wirelessly connectable to the hub, wherein said wearable devices are attachable to parts of a body for collecting health-related data, wherein the wearable devices are allowed to collect data independent of each other, and wherein, when time-synchronized data is required, the hub is configured to send a trigger signal to the wearable devices for initiating and synchronizing measurements.

2. The modular system according to claim 1, wherein the hub is configured to provide clock stimuli to the wearable devices for time synchronization of collected data.

3. The modular system according to claim 1 or 2, wherein the hub is operable in a receive mode, for receiving data from the wearable devices, and in a transmit mode, for transmitting trigger signals to the wearable devices to initiate time synchronized data collection.

4. The modular system according to claim 1, 2 or 3, wherein time-synchronized data is required for computing at least one of a Pulse Arrival Time (PAT) or Pulse Transit Time (PTT) using at least two wearable devices.

5. The modular system according to any one of the preceding claims, wherein time synchronization of data from the wearable devices is achieved through the hub using a master clock that is configured to provide one or more synchronization pulses to the wearable devices.

6. The modular system according to any one of the preceding claims, wherein the hub performs pulsed synchronization of signals in real-time.

7. The modular system according to any one of the preceding claims, wherein the system is configured to provide a bi-directional wireless connectivity between the hub and the wearable devices, wherein the bi-directional wireless connectivity is also used for transmitting and receiving stimuli for synchronizing data collection in real-time.

8. The modular system according to any one of the preceding claims, wherein the hub is usable for recharging devices.

9. The modular system according to any one of the preceding claims, wherein the system comprises a configurational modularity, enabling the detachable connection of a plurality of wearable devices to the hub, each wearable device being configured to wirelessly interface with the hub for bi-directional communication and data transmission, wherein the wearable devices, upon connection to the hub, are operable to autonomously collect health-related data from designated body parts and transmit said data to the hub, further wherein the hub is equipped with synchronization circuitry operable to distribute synchronization signals to the wearable devices for orchestrated data collection and time-aligned data aggregation when time-synchronized measurements are needed.

10. The modular system according to any one of the preceding claims, wherein the wearable devices are equipped with a different sensing mechanisms operable to collect different health-related metrics including cardiovascular data, respiratory data, posture data, and fluid accumulation data, wherein said sensing mechanisms are chosen from a group comprising at least one of: electrocardiogram sensor, photoplethysmogram sensor, heart rate and variability sensor, respiratory rate and/or depth sensor, accelerometer, and bioimpedance sensor, blood glucose level sensor, electrodermal activity sensor, electromyography sensor, electroencephalography sensor or biochemical sensor, and wherein the collected data is wirelessly transmissible to the hub for analysis and/or processing.

11. The modular system according to any one of the preceding claims, wherein the system includes at least an electrocardiogram, ECG, sensor, and a photoplethysmogram, PPG, sensor, wherein the hub is configured to orchestrate time synchronization between the ECG and PPG measurements of the ECG sensor and PPG sensor, respectively.

12. The modular system according to claim 11, wherein the ECG sensor is configured to be positioned on a chest, and the optical PPG sensor is configured to be positioned on another part of the body.

13. A hub configured to wirelessly connect to a plurality of modular wearable devices, each wearable device being attachable to a part of a body for collecting health-related data, wherein said hub comprises a communication module for facilitating independent data collection by each of the wearable devices, and a synchronization module for sending a trigger signal to the wearable devices to initiate and synchronize measurements when time-synchronized data is required, said synchronization module being activated based on predefined conditions or user input.

14. A method of configuring a modular system for collecting health-related data, wherein the method includes providing a hub and modular wearable devices wirelessly connectable to the hub, wherein said wearable devices are attachable to parts of a body for collecting health-related data, wherein the wearable devices are allowed to collect data independent of each other, and wherein, when time-synchronized data is required, the hub is configured to send a trigger signal to the wearable devices for initiating and synchronizing measurements.

15. A method for collecting health-related data using a modular system, the method comprising:
providing a hub;
providing a plurality of modular wearable devices wirelessly connectable to the hub, wherein said wearable devices are attachable to parts of a body;
allowing the wearable devices to collect health-related data independent of each other;
determining whether time-synchronized data collection is required, and in response to determining that time-synchronized data collection is required, sending a trigger signal from the hub to the wearable devices for initiating and synchronizing measurements;
collecting the health-related data from the wearable devices; and
transmitting the collected health-related data to the hub for further processing and/or analysis.
